# EUROPEAN PATENT SPECIFICATION

(11) **EP 2 386 264 B1**
(45) Date of publication and mention of the grant of the patent: **24.06.2020**
(21) Application number: 10016151.2
(22) Date of filing: 29.12.2010
(51) Int. Cl.: A61C 19/06, A61N 5/06

(54) **Mouthpiece that adjusts to user arch sizes and method for making the same**
Mundstück, das sich an die Größe des Benutzerbogens anpasst und Verfahren zur Herstellung desselben
Embout buccal s'ajustant aux tailles d'arcade de l'utilisateur et procédé de fabrication correspondant

(30) Priority: 18.03.2010 US 726770; 11.11.2010 US 943969
(43) Date of publication of application: 16.11.2011
(73) Proprietor: GLO Science, Inc., New York, NY 10018 (US)
(72) Inventor: Levine, Jonathan B., Purchase, NY 10577 (US)
(74) Representative: Pallini Gervasi, Diego

(56) References cited:
- US-A1- 2005 202 363
- US-A1- 2006 078 848
- US-A1- 2007 003 905
- US-A1- 2007 009 856
- US-A1- 2008 003 540

## Description

### BACKGROUND OF THE INVENTION

### Field of the Invention

The present invention relates to a mouthpiece containing lamps to expose electromagnetic radiation to effect oral treatment and, specifically, to aid the activation of an adhesive whitening gel to whiten teeth. The present invention may also be used to kill harmful bacteria in the mouth through the electromagnetic radiation exposure. The mouthpiece adjusts manually to accommodate a broad range of user sizes, yet seals the treatment area from oxygen exposure.

The present disclosure relates generally to a method for effecting an oral treatment using a mouthpiece containing light and heat generating devices and more specifically, to a method for treating teeth by aiding in the activation of a gel applied to the teeth and that contains light and/or heat reactive compounds. Accordingly, the present disclosure relates to a method for whitening teeth, a method for treating gum disease by killing harmful bacteria in the mouth, a method for desensitizing teeth, a method for freshening breath, and a method for accelerating healing of oral sores or ulcers, all through the exposure of the teeth, targeted soft tissue and a specific gel applied thereto, to light and/or heat using a mouthpiece. In the methods, the mouthpiece is designed to adjust manually to accommodate a broad range of user sizes, and closes the system, thus sealing the treatment area from the actives escaping, i.e., oxygen, as well as to adjust during the oral treatment using muscles of the mouth.

### Description of Related Art

Conventional teeth whitening in the dental office takes up to two hours, may be painful and is often costly with noticeable regression beginning in about seven days after the treatment. Improved whitening results are experienced in a professional setting using white light in the 300-990 nm range, but this procedure may be costly and time consuming. Over-the-counter products suffer from other deficiencies, such as difficulty of use, irritation to the fingers and results usually take seven to ten days. Often, there is only minimal improvement. The consumer needs a customizable whitening alternative that yields results similar to the initial professional whitening, but at the convenience of the home that allows for frequency applications and a more stabilized whitening result.

Dentist-supervised tooth whitening involves the controlled use of carbamide or hydrogen peroxide, tailored to a particular patient. Dentists may administer in-office treatments or at-home treatments. Before the tooth whitening treatment, most dentists clean the teeth, fill cavities, and ensure the patient's gums are healthy.

In the document US-A-2007/009856 is revealed a device comprising a main body and a bite surface that extends outward from the main body, where the main body as carrier containing a deformable frame configured to bend under manual force to adjust an orientation of the main body to define an arch configuration. The carrier may also be constructed to emit heat. But the heat source shall be incorporated into the carrier so that the carrier does not ingest the heat source.

In the document US-A-2005/0202363 a dental imaging and treatment system is disclosed, where also LED's as light emitters are arranged within an element as main body. But in this system are not mentioned or showed any heat generators.

In the document US-A-2007/0003905 is explained a tooth whitening apparatus and method with an epoxy handle to be positioned in the mouth of a patient and LED lights for the whitening of the teeth. Pursuant to a heat generation is tolerable to accelerate the chemical reaction.

Most in-office tooth whitening systems use 15 to 35 percent hydrogen peroxide gels, sometimes coupled with a high intensity light to expedite the bleaching chemical reaction.

The in-office procedure involves the dentist gently cleaning the teeth with pumice and then applying a protective barrier on the gums. The dentist then applies hydrogen peroxide paste on the teeth for several minutes, rinses the hydrogen peroxide paste off, and usually reapplies the hydrogen peroxide paste several times. The procedure can achieve about four to six shades of whitening after only one 40-minute treatment.

At-home systems will often use 10 to 20 percent carbamide peroxide gels or up to 6 to 7% hydrogen peroxide that also contain glycerin, carbomer or carbamide, sodium hydroxide, water, and flavoring agents. Some gels that contain more than 10 percent carbamide peroxide will also include sodium fluoride to reduce sensitivity and strengthen teeth.

To begin the at-home procedure, the dentist takes impressions (molds) of the mouth, and then has soft, custom mouth trays made. In administering the treatment, the user places a thin ribbon of the gel into the tray and wears it for two hours during the day, or while sleeping. Most whitening occurs in one to two weeks. In difficult cases, trays may need to be worn for up to six weeks.

A combination of in-office and at-home systems can achieve up to 8 to 10 shades of whitening. Such a procedure is considered safe and effective when monitored by a dentist.

Dentist supervised systems have advantages and disadvantages when compared to over-the-counter tooth whitening products. The main advantage of the dentist supervised system is that the dentist can determine if tooth whitening should be performed and if it will be effective for the patient. Patients with decayed teeth, infected gums, white spots on their teeth, and multiple tooth colored fillings or crowns (caps) on the front teeth may not be good candidates for tooth whitening.

The dentist can also help decide what type of tooth whitening is required (in-office, at-home or both) and the concentration of the whitening gels. The dentist can monitor and treat patients who experience sensitivity to the whitening agents and modify the procedure for those who are having difficulty getting optimal results. Finally, the dentist can help the patient explore porcelain or resin veneers, tooth colored fillings, gum lifts and tooth shaping used with or without tooth whitening. With the help of the dentist, the patient's cosmetic dental goals may be more easily attained.

The disadvantages of dentist supervised whitening systems include higher cost and longer time required to get started when the professional whitening is used. Also, the whitening results will start to regress as early as seven days after the treatment. The in-office and at-home tooth whitening systems can cost between $300-$1,000 (sometimes more). In most cases, at-home systems cost less than the in-office systems. With the dentist supervised systems, there may be a wait to get started. You have to schedule an appointment, wait to be seen and evaluated, and then be treated.

Whitening results are best achieved when there is high frequency of use of the whitening agent, in a safe manner without high concentrations of whitening agents that can bum the gum tissue. By increasing the frequency of the whitening by giving the consumer the ability to whiten at home, the regression of the whitening is greatly reduced or even eliminated.

It is desired to provide a whitening method using a whitening device (mouthpiece) that is coupled with a delivery system of the whitening gel and an adhesive that keeps a photosensitive agent, such as carbamide or hydrogen peroxide, targeted to the area to be whitened, i.e. to the tooth surface. Such a whitening device preferably causes no harmful breakdown by-products, and is hygienically delivered in a single dose.

Further, it is desired to have a whitening method using an adjustable whitening device (mouthpiece) to accommodate a broad range of different size sets of upper or lower teeth of users. It is further desired that the whitening device seal off the area in the mouth to be treated to reduce the amount of oxygen exposure.

In addition, harmful bacteria responsible for causing gum disease in the mouth, specifically, the gram negative anaerobic bacteria, are killed by exposure to oxygen and to ultraviolet light. It would therefore be desirable for a consumer to expose such bacteria to ultraviolet light and to oxygen as well.

With respect to the need to desensitize teeth, tooth sensitivity occurs by the gingiva receding off of the tooth surface and thus exposing the root areas/surfaces of the tooth. These areas have small dentinal tubules that are filled with fluid that originate near the pulp of the tooth and are prone to excitation by certain stimuli of air and cold temperatures. A traditional method to desensitize teeth is to use a toothpaste or dentifrice that contains potassium nitrate and fluoride which seals the tubules and prevents the transmission of the stimulus to the root surface of the tooth.

It is desired to improve the traditional method to desensitize teeth. Even more generally, it is desired to improve the ability to treat teeth in various treatments using a mouthpiece that better conforms to the patient's mouth to thereby enhance the chemical reactions occurring on or around the teeth.

### SUMMARY OF THE INVENTION

One aspect of the invention resides in a mouthpiece as defined in claim 1. Preferred embodiments are defined in the dependent claims. Although not forming part of the present invention, it is also herein disclosed a method for whitening teeth using the intra-oral whitening device or mouthpiece suited to create a whitening and heat effect to increase a reaction rate of a photosensitive agent, such as carbamide or hydrogen peroxide gel. The person whose teeth are to be whitened can wear the device and whiten his/her teeth without the need of a professional office in a safe, effective, convenient and economical fashion.

The mouthpiece of the present invention allows whitening teeth in the convenience of one's home with LED-based white light technology and one or more heat resistors to customize the whitening procedure safely and effectively without the need for a dentist. The mouthpiece adjusts its orientation manually to accommodate a broad range of different size sets of upper or lower teeth of users and to seal a treatment area in the mouth against exposure to oxygen. The mouthpiece also adjusts during treatment as the mouthpiece is warmed by the presence of a heat source therein to enable the mouthpiece to the molded or shaped by the user's oral muscles, e.g., the orbicularis oris.

Also herein disclosed is a method for exposing harmful bacteria in the mouth to oxygen and to ultraviolet light. By doing so, the spread of gum disease caused by the harmful bacteria, such as gram negative anaerobic bacteria, is halted because the oxygen and the ultraviolet light kills the harmful bacteria. Additionally, when the oxygenating compounds are activated, this will have an enhanced effect to kill the anaerobic gram negative bacteria that cause gum disease.

Also herein disclosed is a method for desensitizing teeth by applying a substance containing fluoride and/or potassium nitrate to the teeth, and then utilizing the mouthpiece to generate light and heat thereby causing an increased uptake of fluoride ions and/or potassium nitrate which acts to seal the dentinal tubules.

Also herein disclosed is a method for freshening a patient's breath wherein a freshening gel is applied to the teeth, and then the mouthpiece is placed into the patient's mouth and activated to generate light and heat thereby activating light and/heat reactive compounds that kill the bacteria that cause bad breath.

Also herein disclosed is a method for accelerating healing of sores or ulcers in the mouth wherein a gel containing a compound that will provide such acceleration, e.g., one containing hydrogen peroxide, is applied to the teeth, and then the mouthpiece is placed into the patient's mouth and activated to generate light and heat thereby activating the healing-enhancing compound(s) in the gel, i.e., oxygenating agents, resulting in accelerated healing of the sores or ulcers.

Other aspects of the present disclosure include methods for effecting other oral treatments wherein the application of light and/or heat to the teeth is needed. Accordingly, the present disclosure encompasses the application of a light and/or heat-reactive gel or similar compound, including the gels mentioned above, to teeth with the subsequent placement of the mouthpiece and activation of light and/or heat generators in the mouthpiece to effect an oral treatment.

### BRIEF DESCRIPTION OF THE DRAWING

For a better understanding of the present invention, reference is made to the following description and accompanying drawings, while the scope of the invention is set forth in the appended claims:
FIG. 1 is an isometric view of a dental mouthpiece in accordance with the invention.
FIG. 2 is a partially broken isometric view of the dental mouthpiece of FIG. 1.
FIGS. 3A, 3B and 3C are top views of the dental mouthpiece of FIG. 1 that illustrate how the dental mouthpiece is adjusted to open from the position reflected by FIG. 3A to that of FIG. 3B and to close from the position reflected by FIG. 3A to that of FIG. 3C.
FIG. 4 is a cross-section through a centerline of the dental mouthpiece of FIG. 1.
FIG. 5 is cross-section of the dental mouthpiece of FIG. 1 with respect to a user's tooth.

### DETAILED DESCRIPTION OF THE INVENTION

Described below is a device for use in an oral treatment and several oral treatments are explained in detail. The application of the inventive mouthpiece however is not limited to the oral treatments disclosed herein and encompasses the use of the device for any oral treatment wherein the application of light and/or heat to the oral cavity, e.g., to the teeth and/or gums, is needed. A first exemplifying use of the device is for whitening teeth.

During a teeth whitening treatment in a dental office, a whitening gel is applied to the teeth and a protective barrier is placed on the gums, the mucosa and lips to prevent burning of the tissues by the relatively high concentration of hydrogen peroxide in the whitening gel. A leading edge of the whitening gel is placed on a tooth surface. An LED-based white light is placed a few inches from such a tooth surface to help activate free radical oxygen, most of which becomes lost into the air. By contrast, in this invention, a mouthpiece is provided that seals or encloses a photosensitive agent, such as carbamide or hydrogen peroxide gel, to prevent the loss of the active electrons of the photosensitive agent (carbamide or hydrogen peroxide) into the air.

The mouthpiece holds at least one LED-based white light source, or other comparable light source(s), and preferably at least one heat generator, e.g., a heat resistor that generates heat in response to applied electric current. A power source, which may be remote from the mouthpiece, is in electrical connection with the LED-based white light source(s) via a wire, and the heat generator(s), via the same or different wires. The power source energizes the LED-based white light source(s), which generate light rays, and the heat generator(s), which generates heat. The light rays strike the tooth surface on the front and the edge and the back of the edge while the mouthpiece is in its intended position relative to the tooth surface.

Further, a "closed system" created by the mouthpiece or guard that seals or encloses (against exposure to the atmosphere) is efficient for keeping the active free radical oxygen in close proximity to the teeth to enable their movement onto the tooth surface to breakdown the color pigments inside the tooth. A much lower concentration of the carbamide or hydrogen peroxide gel may be used in comparison to what would be needed to achieve like results in an "open system" that did not seal or enclose the photosensitive agent (carbamide or hydrogen peroxide) from exposure to atmosphere.

Indeed, the whitening device (mouthpiece) of the present invention may be used for seven to ten consecutive days with little to no sensitivity to the teeth and gums. This seven to ten consecutive day use constitutes a higher frequency of use than is available in conventional professional whitening techniques and helps avoid a regression phenomenon that has been observed in the professional whitening technique.

Referring now to the drawings, the mouthpiece **10** adjusts to a broad range of user dental arch sizes (curvature attributed to lower or upper sets of teeth). It also distributes light and heat in a controlled and focused fashion and provides a means of sealing an area being treated from exposure to oxygen.

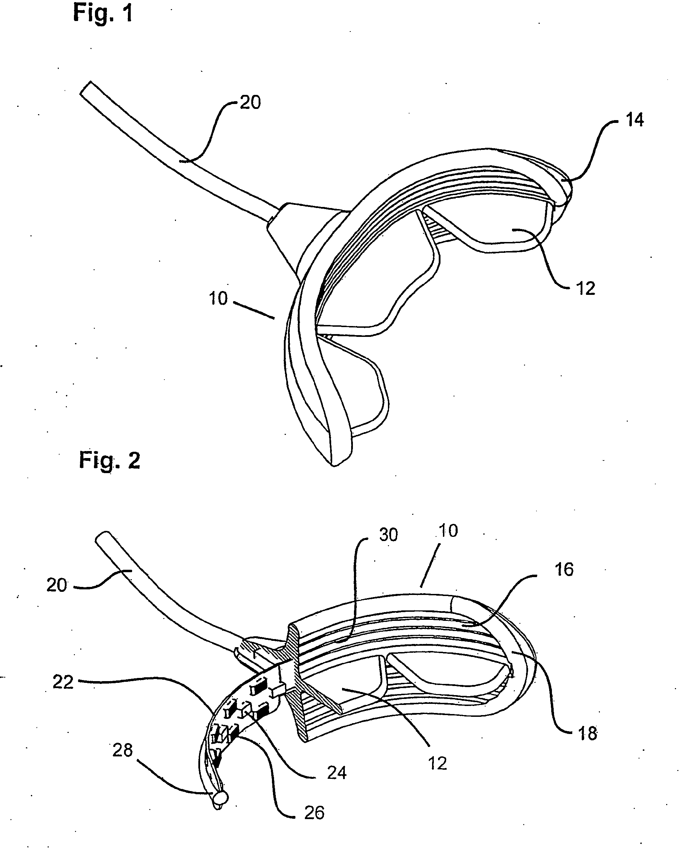

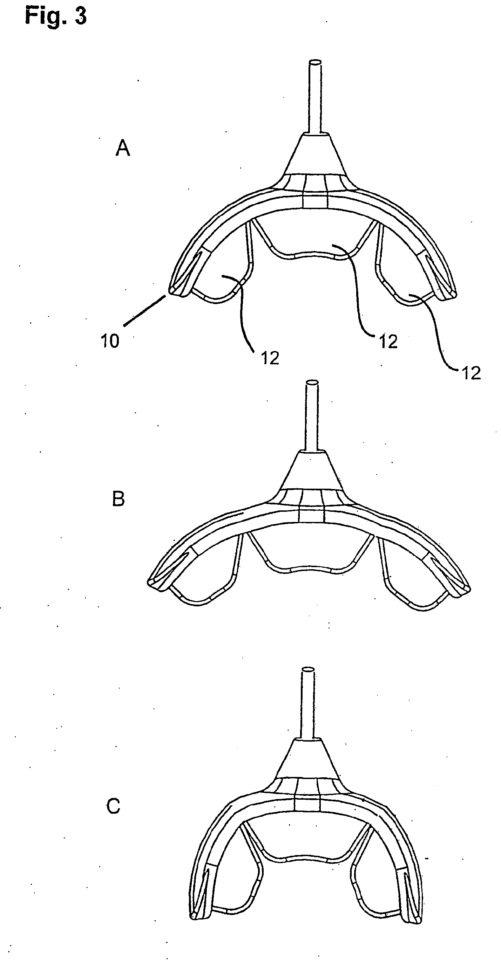

Referring to FIG. 1, the mouthpiece **10** includes a bite surface **12** formed of segments. The bite surface **12** is central and perpendicular to the main body **14**, and thus extends outward from a central portion of the inner surface of the main body **14**, on one side thereof Generally, the bite surface **12** is configured to ensure stability of the mouthpiece **10** when positioned in the user's mouth between the upper and lower arches. As shown, the bite surface **12** is formed from three segmented or separated portions to thereby enable the main body **14** to be bent to conform to the size of the user's mouth and provide the separated bite surface portions with different configurations without overlap of any adjacent bite surface portions. Three such configurations are shown in FIGS. 3A, 3B and 3C. The number of bite surface portions may be different than the three as in the illustrated embodiment.

Referring to FIG. 2, the mouthpiece **10** is preferably formed of a clear, elastomeric, molded main body or outer shape **14** that encases a flexible circuit board **22.** There is a deformable frame **28** that holds the circuit board **22** during fabrication and may be bent by the user to adjust the orientation of the mouthpiece **10** to set the arch for comfort in the user's mouth.

A series of super bright light emitting diodes (LEDs) **24** and heat generators, namely, heat generating resistors **26**, are arrayed on the flexible circuit board **22**, at least the LEDs **24** on an inner, lingual side thereof. A power cord **20** is centrally attached to the outer surface and enables power to be provided to the LEDs **24** and the heat generating resistors **26.** It is envisioned that in some embodiments of the invention, the flexible circuit board **22** includes only a single light emitting diode or other light generating component, and a plurality of light emitting diodes are not present. Similarly, it is envisioned that in some embodiments of the invention, the flexible circuit board **22** includes only a single heat generator, and a plurality of heat generators are not present. Moreover, the heat generating resistors **26** exemplify a heat generator or source inside of the mouthpiece **10** and different components that generate heat from within the mouthpiece **10** to enable the mouthpiece **10** to be warmed and deformed are also envisioned to be within the scope of the appended claims.

A parallel series of textured bands **16**, whose surface texture resembles elongated convex surfaces configured to channel LED light, are formed on the lingual side of the main body **14** for the purposes of LED light diffusion over the surface of the tooth being treated. The textured bands are a form of guided-light optics (GLO) in that they guide or channel light from a source to a desired area of application at which the light causes a reaction of chemicals for a beneficial purpose, in accordance with the invention. Other techniques to guide light from a source to a treatment area are also envisioned within to be within the scope of the appended claims.

Referring to FIG. 3A, areas between the segmented bite surface **12** allow the device to open as in FIG. 3B or close as in FIG. 3C.

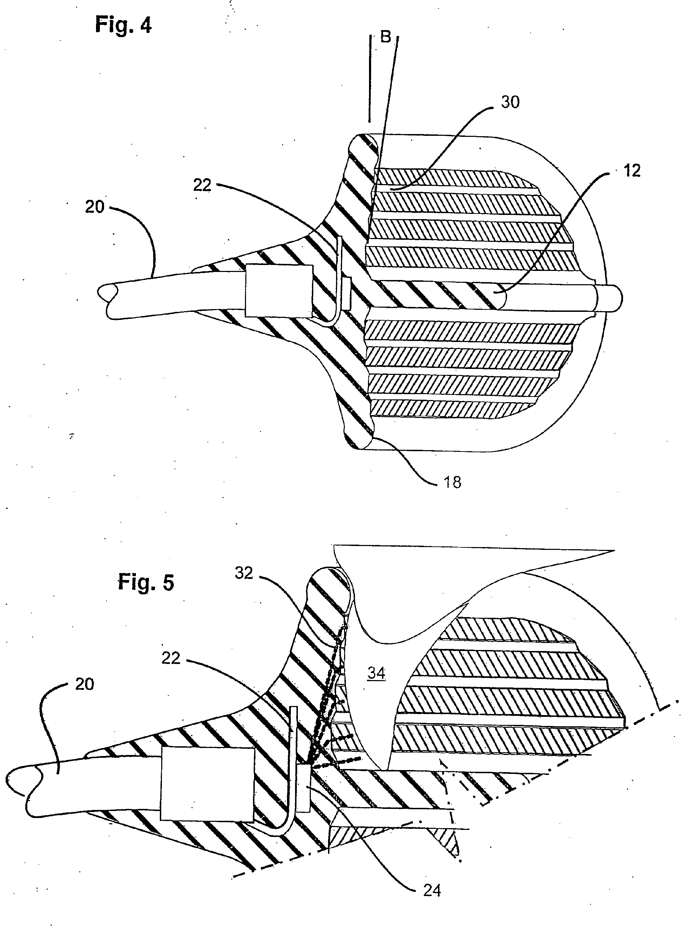

Referring to FIG. 4, an inner surface **30** of the mouthpiece **10** above the bite surface **12** tilts inward at an angle of about 5 to about 15 degrees as noted by B to seal the upper seal bead **18** and borders the upper edge of the mouthpiece **10.** Similarly, an inner surface of the mouthpiece **10** below the bite surface **12** may tilt inward at an angle of about 5 to about 15 degrees to seal the lower seal bead **18** and borders the lower edge of the mouthpiece **10.** The angle for the upper and lower surfaces may be the same or different. The seal beads **18** provide the mouthpiece **10** with rolled and beaded outer edges that form, when the mouthpiece **10** is positioned in the user's mouth against the user's gums, a seal around the mouthpiece **10.**

Referring to FIG. 5, the inward tilt of the inner surface **30** above the bite surface **12** allows the seal bead **18** to contact the gum above the teeth **34.** This contact provides a barrier seal to both retain the whitening gel and to prevent active oxygen from escaping from the treatment area of the tooth (e.g., that area being treated with the whitening gel). The same effect is provided by the inward tilt of the inner surface below the bite surface **12** to the gum below the teeth.

In use, the light **32** emitted by the LEDs **24** is guided using a GLO technique, such as the textured bands 16, and directed to more evenly illuminate the surface of the teeth **34** by the textured bands **16.** The texture of the textured bands **16** provides surfaces that are closer to perpendicular to the light path and less reflective than the generally polished surface of the mouthpiece **10.**

The mouthpiece **10** may be used in various methods for effecting an oral treatment, usually to treat teeth against which the mouthpiece is placed such as by whitening the teeth as mentioned above. In a typical oral treatment, the presence of the electrically activated light emitters, i.e., LEDs **24**, and heat generators, i.e., heat generating resistors **26**, is utilized. The mouthpiece **10** would be positioned in the user's mouth such that the bite surface **12** is between an upper arch and a lower arch and the main body **14** is between a front surface of the teeth and an inner surface of the user's lips. Then, the light emitters and heat generators are electrically activated to effect the oral treatment. To improve the treatment, it would be beneficial for the user to bite down on the bite surface **12** to secure the mouthpiece **10** in their mouth and only thereafter activate the light emitters and heat generators.

When the oral treatment is tooth whitening, a whitening gel would be applied to the user's teeth prior to positioning the mouthpiece **10** in the user's mouth. As noted above, the inwardly tilting inner surfaces of the main body **14** of the mouthpiece cause the seal beads **18** that border an outer edge of the mouthpiece **10** to provide a barrier seal to retain the whitening gel. Thus, when the whitening gel contains hydrogen peroxide, the barrier seal effectively prevents oxygen from escaping an area of the teeth being treated by the hydrogen peroxide-containing whitening gel thereby improving the tooth whitening procedure. Accordingly, it is desired to position the seal beads **18** that are around the outer edge of the mouthpiece **10** against gums of the user (see FIG. 5).

To further improve the oral treatment, the mouthpiece is designed to be anatomically shaped individually for each user. Initially, the main body **14** may be bent via the deformable frame **28** (see FIGS. 3A, 3B and 3C) Thereafter, during treatment when the user is biting down on the bite surface **12**, the mouthpiece **10** can adjust to the user because the heat generating resistors **26** generate heat that warms up material of the main body **14**, e.g., medical grade silicone. At this time, the orbicularis oris muscle of the mouth exerts pressure to mold or shape the now malleable main body **14** to conform to the user's mouth. The bite surface **12**, which may also be formed of medical grade silicone or other comparable material, can also be molded once it is warmed up by the activity of the heat generating resistors **26** A customized fit for the mouthpiece **10** to the user is thereby provided. In addition to or instead of medical grade silicone, bite surface **12** and the main body **14** may be formed of any plasticized material that warms and deforms, i.e., when subjected to heat generated by the heat generating resistors **26**, and ideally maintains its molded form, i.e., after the heat has ceased. Thus, the bite surface **12** and main body **14** will ideally have thermoplastic characteristics. In this manner, during the initial use, the patient would shape the mouthpiece **10** to their mouth, but for subsequent uses, the mouthpiece **10** would maintain that form and shaping would not be substantially required.

The whitening method using mouthpiece **10** provides advantages over prior art whitening techniques, some of which are described above. In an exemplifying professional whitening procedure, a light source is positioned about three inches from the teeth to which the whitening gel has been applied. As a result, only the leading edge of the gel is functioning to whiten the teeth., while the rest of the whitening gel, which breaks down into water and highly reactive oxygen, goes into the atmosphere because the treatment area is not sealed. This type of professional procedure therefore requires about 4 twenty-minute treatments. A consequence of this procedure is a large degree of sensitivity of the teeth, often referred to as zingers. By contrast, use of the mouthpiece **10** in a whitening procedure in accordance with the invention is significantly more efficient taking less time and with less negative consequences to the user because reactive oxygen is prevented from escaping from the treatment area by the incorporation of the light emitting and heat generating systems within the mouthpiece **10** and the presence of the rolled and sealed bead edges **18** formed at the outer edges of the mouthpiece **10.**

Mouthpiece **10** may therefore be used in a method for whitening teeth performed in a home setting allowing a user to whiten their teeth several times in a row spread out over a single day or several days. Moreover, a lower concentration whitening gel, e.g., a 6% to 10.2% whitening hydrogen peroxide gel, may be used for these multiple treatments over several days in a row, in comparison with whitening procedures at dentist's offices wherein an 18% hydrogen peroxide gel is typically used a single time.

Mouthpiece **10** may also be used in a method to desensitize teeth. In a method for desensitizing teeth using mouthpiece **10**, in accordance with the invention, a substance containing fluoride and/or potassium nitrate is applied to the teeth, and then the person puts the mouthpiece **10** into their mouth and activates the LEDs **24** and heat generating resistors **26.** When activated by light and heat generated by the LEDs **24** and heat generating resistors **26**, in the manner described above, the substance causes an increased uptake of fluoride ions and/or potassium nitrate which acts to seal the dentinal tubules. Today, fluoride and potassium nitrate are used in toothpaste to help prevent root sensitivity. However, the user has to brush with this dentifrice multiple times to see a long term effect. With the mouthpiece **10** in combination with this substance, the efficacy of the uptake of these actions is greatly enhanced with the user seeing a much quicker response to desensitizing the root surfaces. The substance can be in the form of a liquid or a gel that can have thixotropic properties that allows for the targeting of the substance onto the exposed root surface with the light and/or heat of the mouthpiece **10.**

Mouthpiece **10** may also be used in a method to accelerate healing of sores or ulcers in the mouth. In such a method, a gel containing a compound that will provide such acceleration, e.g., one containing hydrogen peroxide, is applied to the teeth, and then the mouthpiece **10** is placed into the patient's mouth and activated to generate light and heat, as discussed above, thereby activating the healing-enhancing compound(s) in the gel resulting in accelerated healing of the sores or ulcers. One factor causing the accelerated healing is the better retention of the compounds in the mouth resulting from the ability of the mouthpiece **10** to be warmed and deformed in the patient's mouth which improves the sealing of the mouthpiece **10** to the patient's mouth.

Mouthpiece **10** may also be used in a method for treating gum disease. In such a method, a gel is applied to the gums, the mouthpiece **10** is placed into the patient's mouth and activated to generate light, as discussed above. Harmful bacteria responsible for causing gum disease in the mouth, specifically, the gram negative anaerobic bacteria, are killed by exposure to the light and the active oxygens.

Mouthpiece **10** may also be used in a method for freshening a patient's breath. In this method, a freshening gel is applied to the teeth, and then the mouthpiece is placed into the patient's mouth and activated to generate light and heat thereby activating light and/heat reactive compounds in the gel resulting in breath freshening.

While the foregoing description and drawings represent the preferred embodiments of the present invention, it will be understood that various changes and modifications may be made without departing from the scope of the appended claims.

## Claims

1. A mouthpiece, comprising:
a main body; and
a bite surface (12) that extends outward from the main body (14), the main body (14) containing:
a deformable frame (28) configured to bend under manual force to adjust an orientation of the main body (14) to define an arch configuration; and
at least one light emitter (24) that emits light and at least one heat generator (26) that generates heat; **characterized in that** the main body (14) consists of a material, which is malleable allowing to be molded to conform to the mouth of the user once it is warmed up by the activity of the at least one heat generator (26) and which maintains its molded configuration after said heat generator (26) is deactivated.

2. The mouthpiece of claim 1, wherein the deformable frame (28) holds a flexible circuit board (22), the at least one light emitter (24) and the at least one heat generator (26) being arranged on the flexible circuit board (22).

3. The mouthpiece of claim 2, wherein the main body (14) encases the flexible circuit board (22), the at least one light emitter (24) and the at least one heat generator (26) being on a lingual side of the flexible circuit board (22).

4. The mouthpiece of claim 2, further comprising a power cord (20) attached to a central region of an outside facing surface of the flexible circuit board (22).

5. The mouthpiece of claim 1, wherein the at least one light emitter (24) comprises at least one light emitting diode.

6. The mouthpiece of claim 1, wherein the deformable frame (28) holds a flexible circuit board (22), the at least one light emitter (24) comprising a plurality of light emitting diodes and the at least one heat generator (26) comprising a plurality of heat generating resistors arranged in an array on the flexible circuit board (22).

7. The mouthpiece of claim 1, further comprising a parallel series of textured bands (16) on a lingual side of the main body (14) to diffuse light from the light emitter (24) toward areas to be treated.

8. The mouthpiece of claim 7, wherein the mouthpiece includes a polished surface, the textured bands (16) are arranged to guide a direct the light from the at least one light emitter (24) so as to more evenly illuminate surfaces of the teeth than would otherwise be the case without the textured bands (16) being present, the textured bands (16) having a surface texture that is closer to being perpendicular to a path of the light and less reflective than the polished surface of the mouthpiece.

9. The mouthpiece of claim 1, wherein the bite surface (12) is segmented into a plurality of spaced apart segments that allow room for the deformable frame to flex to selectively re-orient a configuration of the main body (14) between open and closed positions.

10. The mouthpiece of claim 1, wherein the mouthpiece (10) has an inner surface that inwardly tilts within an angular range of 5 to 15 degrees to seal a seal bead (18), which borders an edge of the mouthpiece.

11. The mouthpiece of claim 10, wherein the inner surface inwardly tilts to allow the seal bead (18) to contact a gum above a tooth, the contact providing a barrier seal to both retain a whitening gel and to prevent oxygen from entering an area of the tooth to be treated by the whitening gel.

12. A method for creating a closed oral treatment system with a customized fit, comprising:
providing a deformable mouthpiece (10) having at least one electrically activated light emitter (24) and at least one electrically activated heat generator (26);
positioning the mouthpiece (10) in a user's mouth; and
electrically activating the at least one light emitter (24) and the at least one heat generator (26), once it is warmed up by the activity of the at least one heat generator (26) to cause the mouthpiece (10) to be deformable by the user such that the mouthpiece (10) conforms to the user's mouth to enable the user to form a seal proximate to the user's teeth such that free radical oxygen remains in close proximity to the user's teeth.

13. The method of claim 12, wherein at least a portion of the mouthpiece (10) is made of thermoplastic material.

14. The method of claim 12, wherein the mouthpiece (10) has a main body (14) having a seal bead around an outer edge and a bite surface that extends outward from the main body (14), the mouthpiece (10) being positioned in the user's mouth such that the bite surface is between an upper arch and a lower arch, the main body (104) is between a front surface of the teeth and an inner surface of the user's lips, and the seal bead is against gums of the user.

15. The method of claim 14, further comprising arranging the at least one heat generator (26) in the main body (14) to thereby cause the main body (14) to warm up when the at least one heat generator (26) is activated.

## Patentansprüche

1. Ein Mundstück, Folgendes umfassend:
einen Hauptkörper; und
eine Bissfläche (12), die sich vom Hauptkörper (14) nach außen erstreckt, wobei der Hauptkörper (14) Folgendes enthält:
einen verformbaren Rahmen (28), der so ausgebildet ist, dass er sich mit Hilfe von manueller Kraft biegen kann, um eine Orientierung des Hauptkörpers (14) einzustellen, um eine Bogenkonfiguration zu definieren; und
mindestens einen Lichtemitter (24), der Licht ausstrahlt, und mindestens einen Wärmeerzeuger (26),
der Wärme erzeugt; **dadurch gekennzeichnet, dass** der Hauptkörper (14) aus einem Werkstoff besteht, der plastisch verformbar ist, so dass er so geformt werden kann, dass er sich an den Mund des Benutzers anpasst, wenn er durch die Aktivität des mindestens einen Wärmeerzeugers (26) erwärmt wird, und der seine geformte Konfiguration nach der Deaktivierung des Wärmeerzeugers (26) beibehält.

2. Mundstück gemäß Anspruch 1, wobei der verformbare Rahmen (28) eine flexible Leiterplatte (22) hält, wobei der mindestens eine Lichtemitter (24) und der mindestens eine Wärmeerzeuger (26) auf der flexiblen Leiterplatte (22) angeordnet sind.

3. Mundstück gemäß Anspruch 2, bei dem der Hauptkörper (14) die flexible Leiterplatte (22) umschließt, wobei sich der mindestens eine Lichtemitter (24) und der mindestens eine Wärmeerzeuger (26) auf einer lingualen Seite der flexiblen Leiterplatte (22) befinden.

4. Mundstück gemäß Anspruch 2, das ferner ein Netzkabel (20) umfasst, das an einem zentralen Bereich einer nach außen gerichteten Oberfläche der flexiblen Leiterplatte (22) befestigt ist.

5. Mundstück gemäß Anspruch 1, wobei der mindestens eine Lichtemitter (24) mindestens eine Leuchtdiode umfasst.

6. Mundstück gemäß Anspruch 1, wobei der verformbare Rahmen (28) eine flexible Leiterplatte (22) hält, wobei der mindestens eine Lichtemitter (24) eine Vielzahl von Leuchtdioden und der mindestens eine Wärmeerzeuger (26) eine Vielzahl von wärmeerzeugenden Widerständen umfasst, die in einer Anordnung auf der flexiblen Leiterplatte (22) angeordnet sind.

7. Mundstück gemäß Anspruch 1, das weiterhin eine parallele Reihe von texturierten Bändern (16) auf einer lingualen Seite des Hauptkörpers (14) umfasst, um Licht vom Lichtemitter (24) in Richtung der zu behandelnden Bereiche zu streuen.

8. Mundstück gemäß Anspruch 7, wobei das Mundstück eine polierte Oberfläche einschließt, wobei die texturierten Bänder (16) so angeordnet sind, dass sie eine direkte das Licht von dem mindestens einen Lichtemitter (24) führen, so dass die Oberflächen der Zähne gleichmäßiger beleuchtet werden, als es ohne die texturierten Bänder (16) der Fall wäre, wobei die texturierten Bänder (16) eine Oberflächentextur aufweisen, die eher senkrecht zu einem Weg des Lichts und weniger reflektierend als die polierte Oberfläche des Mundstücks ist.

9. Mundstück gemäß Anspruch 1, wobei die Bissfläche (12) in eine Vielzahl von beabstandeten Segmenten segmentiert ist, die dem verformbaren Rahmen Raum zur Biegung geben, um eine Konfiguration des Hauptkörpers (14) zwischen einer offenen und einer geschlossenen Position selektiv umzuorientieren.

10. Mundstück gemäß Anspruch 1, wobei das Mundstück (10) eine Innenfläche aufweist, die innerhalb eines Winkelbereichs von 5 bis 15 Grad nach innen geneigt ist, um einen Dichtungswulst (18) abzudichten, der einen Rand des Mundstücks begrenzt.

11. Mundstück gemäß Anspruch 10, wobei die Innenfläche nach innen geneigt ist, damit der Dichtungswulst (18) mit dem Zahnfleisch über einem Zahn in Kontakt kommen kann, wobei der Kontakt eine Barrieredichtung bereitstellt, um sowohl ein Bleichgel zurückzuhalten als auch zu verhindern, dass Sauerstoff in einen Bereich des Zahns, der durch das Bleichgel behandelt werden soll, eindringt.

12. Verfahren zur Erzeugung eines geschlossenen Mundbehandlungssystems mit einer maßgeschneiderten Passform, Folgendes umfassend:
Bereitstellen eines verformbaren Mundstücks (10) mit mindestens einem elektrisch aktivierten Lichtemitter (24) und mindestens einem elektrisch aktivierten Wärmeerzeuger (26); Positionieren des Mundstücks (10) im Mund eines Benutzers; und
elektrische Aktivierung des mindestens einen Lichtemitters (24) und des mindestens einen Wärmeerzeugers (26), sobald es durch die Aktivität des mindestens einen Wärmeerzeugers (26) erwärmt wird, um zu bewirken, dass das Mundstück (10) durch den Benutzer verformbar ist, so dass das Mundstück (10) sich an den Mund des Benutzers anpasst, um es dem Benutzer zu ermöglichen, eine Dichtung in der Nähe der Zähne des Benutzers zu bilden, so dass der Sauerstoff als freies Radikal in unmittelbarer Nähe der Zähne des Benutzers bleibt.

13. Verfahren gemäß Anspruch 12, wobei mindestens ein Teil des Mundstücks (10) aus thermoplastischem Werkstoff hergestellt ist.

14. Verfahren gemäß Anspruch 12, wobei das Mundstück (10) einen Hauptkörper (14) mit einem Dichtungswulst um eine Außenkante und eine Bissfläche aufweist, die sich von dem Hauptkörper (14) nach außen erstreckt, wobei das Mundstück (10) in dem Mund des Benutzers so positioniert ist, dass die Bissfläche zwischen einem oberen Bogen und einem unteren Bogen liegt, der Hauptkörper (104) zwischen einer Vorderfläche der Zähne und einer Innenfläche der Lippen des Benutzers liegt und der Dichtungswulst gegen das Zahnfleisch des Benutzers anliegt.

15. Verfahren gemäß Anspruch 14, ferner umfassend das Anordnen des mindestens einen Wärmeerzeugers (26) im Hauptkörper (14), um dadurch den Hauptkörper (14) zu erwärmen, wenn der mindestens eine Wärmeerzeuger (26) aktiviert wird.

## Revendications

1. Embout buccal, comprenant :
un corps principal ; et
une surface à mordre (12) qui s'étend vers l'extérieur à partir du corps principal (14), le corps principal (14) contenant :
une armature déformable (28) configurée pour se plier sous une force manuelle pour régler une orientation du corps principal (14) pour définir une configuration d'arcade ; et
au moins un émetteur de lumière (24) qui émet une lumière et au moins un générateur de chaleur (26)
qui génère de la chaleur ; **caractérisé en ce que** le corps principal (14) est constitué d'un matériau qui est malléable lui permettant d'être moulé pour se conformer à la bouche de l'utilisateur une fois qu'il est échauffé par l'activité de l'au moins un générateur de chaleur (26) et qui conserve sa configuration moulée après que ledit générateur de chaleur (26) a été désactivé.

2. Embout buccal selon la revendication 1, dans lequel l'armature déformable (28) contient une carte de circuits souple (22), l'au moins un émetteur de lumière (24) et l'au moins un générateur de chaleur (26) étant agencés sur la carte de circuits souple (22).

3. Embout buccal selon la revendication 2, dans lequel le corps principal (14) renferme la carte de circuits souple (22), l'au moins un émetteur de lumière (24) et l'au moins un générateur de chaleur (26) étant sur un côté lingual de la carte de circuits souple (22).

4. Embout buccal selon la revendication 2, comprenant en outre un cordon d'alimentation (20) attaché à une région centrale d'une surface faisant face vers l'extérieur de la carte de circuits souple (22).

5. Embout buccal selon la revendication 1, dans lequel l'au moins un émetteur de lumière (24) comprend au moins une diode électroluminescente.

6. Embout buccal selon la revendication 1, dans lequel l'armature déformable (28) contient une carte de circuits souple (22), l'au moins un émetteur de lumière (24) comprenant une pluralité de diodes électroluminescentes et l'au moins un générateur de chaleur (26) comprenant une pluralité de résistances de génération de chaleur agencées selon un réseau sur la carte de circuits souple (22).

7. Embout buccal selon la revendication 1, comprenant en outre une série parallèle de bandes texturées (16) sur un côté lingual du corps principal (14) pour diffuser une lumière provenant de l'émetteur de lumière (24) en direction de zones à traiter.

8. Embout buccal selon la revendication 7, dans lequel l'embout buccal inclut une surface polie, les bandes texturées (16) sont agencées pour guider et diriger la lumière provenant de l'au moins un émetteur de lumière (24) de façon à éclairer de manière plus uniforme des surfaces des dents que ce ne serait autrement le cas sans que les bandes texturées (16) soient présentes, les bandes texturées (16) ayant une texture de surface qui est plus proche d'être perpendiculaire à un trajet de la lumière et moins réfléchissante que la surface polie de l'embout buccal.

9. Embout buccal selon la revendication 1, dans lequel la surface à mordre (12) est segmentée en une pluralité de segments espacés qui laissent de la place pour que l'armature déformable se fléchisse pour réorienter sélectivement une configuration du corps principal (14) entre des positions ouverte et fermée.

10. Embout buccal selon la revendication 1, dans lequel l'embout buccal (10) possède une surface interne qui s'incline vers l'intérieur au sein d'une plage angulaire de 5 à 15 degrés pour rendre étanche un cordon d'étanchéité (18), qui borde un bord de l'embout buccal.

11. Embout buccal selon la revendication 10, dans lequel la surface interne s'incline vers l'intérieur pour permettre au cordon d'étanchéité (18) d'être en contact avec une gencive au-dessus d'une dent, le contact fournissant un joint d'étanchéité barrière pour à la fois retenir un gel de blanchiment et empêcher l'oxygène de pénétrer dans une zone de la dent à traiter par le gel de blanchiment.

12. Procédé pour créer un système de traitement oral fermé avec un ajustement personnalisé, consistant à :
fournir un embout buccal déformable (10) ayant au moins un émetteur de lumière électriquement activé (24) et au moins un générateur de chaleur électriquement activé (26) ; positionner l'embout buccal (10) dans la bouche d'un utilisateur ; et
activer électriquement l'au moins un émetteur de lumière (24) et l'au moins un générateur de chaleur (26), une fois qu'il est échauffé par l'activité de l'au moins un générateur de chaleur (26) pour amener l'embout buccal (10) à être déformable par l'utilisateur de sorte que l'embout buccal (10) se conforme à la bouche de l'utilisateur pour permettre à l'utilisateur de former un joint d'étanchéité à proximité des dents de l'utilisateur de sorte que des radicaux libres d'oxygène restent à proximité immédiate des dents de l'utilisateur.

13. Procédé selon la revendication 12, dans lequel au moins une partie de l'embout buccal (10) est constituée de matériau thermoplastique.

14. Procédé selon la revendication 12, dans lequel l'embout buccal (10) possède un corps principal (14) ayant un cordon d'étanchéité autour d'un bord externe et une surface à mordre qui s'étend vers l'extérieur à partir du corps principal (14), l'embout buccal (10) étant positionné dans la bouche de l'utilisateur de sorte que la surface à mordre est entre une arcade supérieure et une arcade inférieure, le corps principal (104) est entre une surface avant des dents et une surface interne des lèvres de l'utilisateur, et le cordon d'étanchéité est contre les gencives de l'utilisateur.

15. Procédé selon la revendication 14, consistant en outre à agencer l'au moins un générateur de chaleur (26) dans le corps principal (14) pour amener ainsi le corps principal (14) à s'échauffer lorsque l'au moins un générateur de chaleur (26) est activé.
